(19) Europäisches Patentamt
European Patent Office
Office européen des brevets

(11) Veröffentlichungsnummer: **0 007 399**
**B1**

(12) # EUROPÄISCHE PATENTSCHRIFT

(45) Veröffentlichungstag der Patentschrift: **20.01.82**

(21) Anmeldenummer: **79101662.9**

(22) Anmeldetag: **30.05.79**

(51) Int. Cl.³: **C 07 D 401/06,** A 61 K 31/40, A 61 K 31/445 // (C07D401/06, 209/04, 211/08)

(54) **Indolalkylamine, diese enthaltende pharmazeutische Zubereitungen und Verfahren zu ihrer Herstellung.**

(30) Priorität: **24.06.78 DE 2827874**
**16.03.79 DE 2910367**

(43) Veröffentlichungstag der Anmeldung: **06.02.80 Patentblatt 80/3**

(45) Bekanntmachung des Hinweises auf die Patenterteilung: **20.01.82 Patentblatt 82/3**

(84) Benannte Vertragsstaaten: **AT BE CH DE FR GB IT NL SE**

(56) Entgegenhaltungen:
**DE-A-2 060 816**
**GB-A-921 507**
**US-A-3 494 920**
**US-A-4 021 431**
**US-A-3 217 011**
**US-A-3 238 215**

(73) Patentinhaber: **Merck Patent Gesellschaft mit beschränkter Haftung, Frankfurter Strasse 250, D-6100 Darmstadt (DE)**

(72) Erfinder: **Hausberg, Hans-Heinrich, Dr., Hahlgartenstrasse 12, D-6108 Weiterstadt 1 (DE)**
Erfinder: **Koppe, Volker, Dr., Kollwitzweg 42, D-6100 Darmstadt (DE)**
Erfinder: **Poetsch, Eike, Dr., Am Buchwald 4, D-6109 Mühltal-Waschenbach (DE)**
Erfinder: **Saiko, Otto, Dr., Mozartweg 14, D-6100 Darmstadt (DE)**
Erfinder: **Seyfried, Christoph, Dr., Am Landbach 9, D-6104 Seeheim-Jugenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

Indolalkylamine' diese enthaltende pharmazeutische Zubereitungen und Verfahren zu ihrer Herstellung

Die Erfindung betrifft neue Indolalkylamine der allgemeinen Formel I

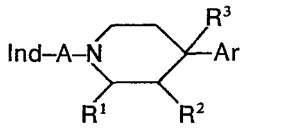

I

worin

Ind einen 1-$R^4$-2-$R^5$-Indol-3-ylrest, der im Benzolring ein- bis dreifach durch Alkyl, O-Alkyl, S-Alkyl, OH, F, Cl, Br, $CF_3$ und/oder CN substituiert sein kann,

A $-(CH_2)_4-$, $-(CH_2)_3-CO-$ oder $-CO-(CH_2)_2-CO-$,

$R^1$ H oder Methyl,

$R^2$ H oder zusammen mit $R^3$ eine C–C-Bindung,

$R^3$ H oder zusammen mit $R^2$ eine C–C-Bindung,

$R^4$ und $R^5$ jeweils H, Alkyl oder Phenyl und

Ar eine unsubstituierte oder ein- oder zweifach durch Alkyl, F, Cl, Br, J und/oder $CF_3$ substituierte Phenylgruppe

bedeuten und

worin die Alkylgruppen jeweils 1–4 C-Atome besitzen, sowie deren physiologisch unbedenklichen Säureadditionssalze.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, dass die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem dopamin-stimulierende und serotonin-aufnahmehemmende (Anti-Parkinson- und antidepressive) Wirkungen. Im einzelnen induzieren die Verbindungen der Formel I contralaterales Drehverhalten in Hemiparkinson-Ratten (feststellbar nach der Methode von Ungerstedt et al., Brain Res. 24, (1970), 485–493) und vermindern den Dopamin-Umsatz im Gehirn (feststellbar an Ratten nach der Methode von Anden et al., Europ. J. Pharmacol. 11, (1970), 303–314). Zusätzlich hemmen die Verbindungen die Aufnahme von Serotonin in Synaptosomen in vitro (feststellbar an Ratten nach Kannengiesser et al., Biochem. Pharmacol. 22, (1973), 73–84) und hemmen in vivo die durch Tyraminderivate induzierte Serotonin-Freisetzung im Gehirn (feststellbar an Ratten nach Carlsson et al., Europ. J. Pharmacol. 5, (1969), 357–366; 367–373). Weiterhin treten analgetische Wirkungen auf.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Indolalkylamine der Formel I sowie ihre physiologisch unbedenklichen Säureadditionssalze.

In den Resten Ind, $R^4$, $R^5$ und Ar bedeutet Alkyl vorzugsweise Methyl, ferner auch Äthyl, n-Propyl, Isopropyl, n-Butyl, Isobutyl, sek.-Butyl oder tert.-Butyl. O-Alkyl ist vorzugsweise Methoxy, ferner auch Äthoxy, n-Propoxy, Isopropoxy, n-Butoxy, Isobutoxy, sek.-Butoxy oder tert.-Butoxy. S-Alkyl steht vorzugsweise für Methylthio, aber auch für Äthylthio, n-Propylthio, Isopropylthio, n-Butylthio, Isobutylthio, sek.-Butylthio oder tert.-Butylthio.

Der Rest Ind bedeutet insbesondere einen unsubstituierten Indol-3-yl-rest. Falls Ind jedoch einen substituierten Indol-3-ylrest bedeutet, so ist er vorzugsweise einfach, insbesondere in der 1-, 2- oder 5-Stellung substituiert. Weiterhin ist eine Substitution in 4-, 6- oder 7-Stellung möglich. Bevorzugte disubstituierte Indol-3-yl-reste sind in 1,2-, 1,5-, 2,5-, 4,7-, 5,6- oder 5,7-Stellung substituiert; Disubstitution ist auch in 1,4-, 1,6-, 1,7-, 2,4-, 2,6-, 2,7-, 4,5-, 4,6- oder 6,7-Stellung möglich. In allen diesen Fällen können die Substituenten gleich oder verschieden sein. Bevorzugte trisubstituierte Indol-3-ylreste sind in 1,2,5-, 4,5,6- oder 5,6,7-Stellung substituiert; Trisubstitution ist auch in 1,2,4-, 1,2,6-, 1,2,7-, 1,4,5-, 1,4,6-, 1,4,7-, 1,5,6-, 1,5,7-, 1,6,7-, 2,4,5-, 2,4,6-, 2,4,7-, 2,5,6-, 2,5,7-, 2,6,7-, 4,5,7- oder 4,6,7-Stellung möglich.

Weiterhin kommt Tetrasubstitution des Indolrings in 1,2,4,5-, 1,2,4,6-, 1,2,4,7-, 1,2,5,6-, 1,2,5,7-, 1,2,6,7-, 1,4,5,6-, 1,4,5,7-, 1,4,6,7-, 1,5,6,7-, 2,4,5,6-, 2,4,5,7-, 2,4,6,7- oder 2,5,6,7-Stellung, Pentasubstitution in 1,2,4,5,6-, 1,2,4,5,7-, 1,2,4,6,7- oder 1,2,5,6,7-Stellung in Betracht.

Im einzelnen sind die bevorzugten Substituenten im Benzolring des Restes Ind Methyl, Äthyl, Methoxy- Äthoxy, Methylthio, Äthylthio, OH, F, Cl, Br, $CF_3$ und CN. $R^4$ ist bevorzugt H oder Methyl, $R^5$ ist bevorzugt H, Methyl oder Phenyl. Einige bevorzugte Bedeutungen des Restes Ind sind dementsprechend Indol-3-yl, ferner 1-, 2-, 4-, 5-, 6- oder 7-Methylindol-3-yl, 1-, 2-, 4-, 5-, 6- oder 7-Äthyl-indol-3-yl, 1- oder 2-Phenylindol-3-yl, 4-, 5-, 6- oder 7-Methoxyindol-3-yl, 4-, 5-, 6- oder 7-Äthoxyindol-3-yl, 4-, 5-, 6- oder 7-Methylthioindol-3-yl, 4-, 5-, 6- oder 7-Äthylthioindol-3-yl, 4-, 5-, 6- oder 7-Fluorindol-3-yl, 4-, 5-, 6- oder 7-Chlorindol-3-yl, 4-, 5-, 6- oder 7-Bromindol-3-yl, 4-, 5-, 6- oder 7-Trifluormethyl-indol-3-yl, 4-, 5-, 6- oder 7-Cyanindol-3-yl, 1,2-, 1,4-, 1,5-, 1,6-, 1,7-, 2,4-, 2,5-, 2,6-, 2,7-, 4,5-, 4,6-, 4,7-, 5,6-, 5,7- oder 6,7-Dimethylindol-3-yl, 1-Methyl-2-phenylindol-3-yl, 2-Phenyl-4-, -5-, -6- oder -7-methylindol-3-yl, 1-Methyl-4-, -5-, -6-oder -7-methoxyindol-3-yl, 1-Methyl-4-, -5-, -6- oder -7-fluorindol-3-yl, 1-Methyl-4-, -5-, -6- oder -7-chlorindol-3-yl, 1-Methyl-4-, -5-, -6- oder -7-bromindol-3-yl, 1-Methyl-4-, -5-, -6- oder -7-trifluormethylindol-3-yl, 1-Methyl-4-, -5-, -6- oder -7-cyanindol-3-yl, 2-Methyl-4-, -5-, -6-oder -7-methoxyindol-3-yl, 2-Methyl-4-, -5-, -6- oder -7-methylthioindol-3-yl, 2-Methyl-4-, -5-, -6- oder -7-fluorindol-3-yl, 2-Methyl-4-, -5-, -6- oder -7-chlorindol-3-yl, 2-Methyl-4-, -5-, -6- oder -7-bromindol-3-yl, 2-Methyl-4-, -5-, -6- oder -7-trifluormethylindol-3-yl, 2-Methyl-4-, -5-, -6- oder -7-cyanindol-3-yl, 4-Methyl-5-fluorindol-3-yl, 5-Fluor-6- oder -7-methylindol-3-yl, 4-Methyl-5-chlorindol-3-yl, 4-Chlor-5-methylindol-3-yl, 5-Methyl-6- oder 7-chlorindol-3-yl, 5-Chlor-6- oder -7-methylindol-3-yl, 4,5-, 4,6-, 4,7-, 5,6-, 5,7- oder 6,7-Dimethoxyindol-3-yl, 4,5- 4,6-, 4,7-, 5,6-, 5,7- oder 6,7-Dichlorindol-3-yl, 4-Trifluormethyl-5-, -6- oder -7-chlorindol-3-yl, 1,2,4-, 1,2,5-, 1,2,6-, 1,2,7-, 1,4,5-, 1,4,6-, 1,4,7-, 1,5,6-, 1,5,7-, 1,6,7-, 2,4,5-, 2,4,6-, 2,4,7-, 2,5,6-, 2,5,7- oder 2,6,7-Trimethylindol-3-yl, 4,5,6-, 4,5,7-, 4,6,7- oder 5,6,7-Trimethoxyindol-3-yl, 4,5,6-, 4,5,7-, 4,6,7- oder 5,6,7-Trichlorindol-3-yl.

Die Gruppe A ist bevorzugt $-(CH_2)_4-$.

$R^1$ ist bevorzugt H. $R^2$ und $R^3$ sind bevorzugt zusammen eine C–C-Bindung.

Der Rest Ar ist bevorzugt unsubstituiertes Phenyl. Falls Ar eine substituierte Phenylgruppe bedeutet, so ist diese vorzugsweise einfach substituiert. Sie kann jedoch auch zweifach substituiert sein, wobei die Substituenten gleich oder verschieden sein können. Bevorzugte Substituenten an der Phenylgruppe sind Methyl, F, Cl, Br und Trifluormethyl. Im einzelnen ist Ar bevorzugt Phenyl, o-, m- oder p-Fluorphenyl, o-, m- oder p-Chlorphenyl, o-, m- oder p-Bromphenyl, o-, m- oder p-Tolyl, o-, m- oder p-Trifluormethylphenyl, ferner z.B. o-, m- oder p-Äthylphenyl, o-, m- oder p-n-Propylphenyl, o-, m- oder p-Isopropylphenyl, o-, m- oder p-n-Butylphenyl, o-, m- oder p-Isobutylphenyl, o-, m- oder p-Jodphenyl, weiterhin Dihalogenphenyl wie 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Fluor-4-chlorphenyl, 2-Brom-4-chlorphenyl; Dimethylphenyl wie 2,3-, 2,4-, 2,5-, 2,6- 3,4- oder 3,5-Dimethylphenyl; Methyl-chlorphenyl wie 2-Methyl-4-chlorphenyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ie ausgedrückt werden, die der Formel I entsprechen und worin die nicht näher bezeichneten Reste die bei Formel I angegebene Bedeutung haben, worin jedoch

| | | |
|---|---|---|
| in Ia | Ind | Indol-3-yl, 1-Methyl-indol-3-yl oder 2-Methylindol-3-yl bedeutet; |
| in Ib | A | $-(CH_2)_4-$ bedeutet; |
| in Ic | $R^1$ | H bedeutet; |
| in Ie | $R^2$ und $R^3$ | zusammen eine C–C-Bindung bedeuten; |
| in If | Ar | Phenyl, p-Fluorphenyl, o- oder p-Chlorphenyl, o- oder p-Bromphenyl, p-Tolyl oder m-Trifluormethylphenyl bedeutet; |
| in Ig | Ind | Indol-3-yl, 1-Methylindol-3-yl oder 2-Methylindol-3-yl, |
| | A | $-(CH_2)_4-$ und |
| | Ar | Phenyl, p-Chlorphenyl oder m-Trifluormethylphenyl bedeuten; |
| in Ih | Ind | Indol-3-yl, 1-Methylindol-3-yl oder 2-Methylindol-3-yl, |
| | A | $-(CH_2)_4-$ und |
| | Ar | Phenyl bedeuten. |

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrere Racemate sowie in verschiedenen optisch-aktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, dass man eine Verbindung der allgemeinen Formel II

$$Ind\text{-}A\text{-}X^1 \qquad II$$

worin $X^1$ X oder $NH_2$ und
X Cl, Br, J, OH oder eine reaktionsfähig funktionell abgewandelte OH-Gruppe bedeuten und
Ind und A die angegebenen Bedeutungen haben,
mit einer Verbindung der allgemeinen Formel III

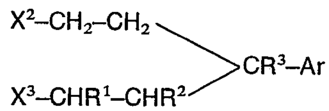
III

worin
$X^2$ und $X^3$ gleich oder verschieden sein können und falls $X^1 = NH_2$ ist, jeweils X, andernfalls zusammen NH bedeuten und
$R^1$, $R^2$, $R^3$ und Ar die angegebenen Bedeutungen haben
umsetzt
oder dass man ein Isonitril der allgemeinen Formel IV

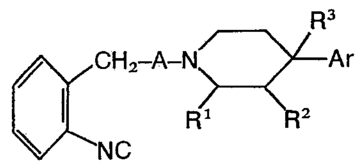
IV

worin
der Benzolring ein- bis dreifach durch Alkyl, O-Alkyl, S-Alkyl, OH, F, Cl, Br, $CF_3$ und/oder CN substituiert sein kann und
$R^1$, $R^2$, $R^3$, A und Ar die angegebenen Bedeutungen haben
mit Hilfe von Lithium-2,2,6,6-tetramethylpiperidid cyclisiert
oder dass man ein Pyridiniumsalz der allgemeinen Formel V

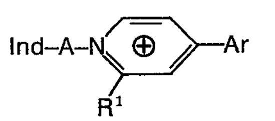
$An^{\ominus}$ V

worin
$An^{\ominus}$ ein Anion einer starken Säure bedeutet und
Ind, A, $R^1$ und Ar die angegebenen Bedeutungen haben
mit einem reduzierenden Mittel behandelt
und/oder dass man gegebenenfalls in einer Verbindung der Formel I eine oder zwei CO-Gruppen zu $CH_2$-Gruppen reduziert und/oder eine Doppelbindung hydriert und/oder eine freie NH-Gruppe im Indolring alkyliert und/oder eine Alkoxygruppe unter Bildung einer Hydroxygruppe spaltet und/oder dass man eine Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New. York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II bis V können gewünschtenfalls auch in situ gebildet werden, derart, dass man sie aus dem Reaktionsgemisch nicht isoliert, sondern sofort weiter zu den Verbindungen der Formel I umsetzt.

Die Verbindungen der Formel I werden vorzugsweise durch Umsetzung von Indolderivaten der Formel II mit Verbindungen der Formel III erhalten.

In den Indolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br., er kann jedoch auch J, OH oder eine reaktionsfähig funktionell abgewandelte OH-Gruppe bedeuten, insbesondere Alkylsulfonyloxy mit 1–6 (z.B. Methansulfonyloxy) oder Arylsulfonyloxy mit 6–10 C-Atomen (z.B. Benzolsulfonyloxy, p-Toluolsulfonyloxy, 1- oder 2-Naphthalin-sulfonyloxy).

Dementsprechend sind die Indolalkylamine der Formel I insbesondere durch Umsetzung der Verbindungen der Formeln Ind-A-Cl oder Ind-A-Br mit Piperidinderivaten der Formel III, worin $X^2$ und $X^3$ zusammen eine NH-Gruppe bedeuten (nachstehend als IIIa bezeichnet) erhältlich.

Die Verbindungen der Formeln II und III sind zum Teil bekannt; die nicht bekannten Verbindungen der Formeln II und III können leicht analog zu den bekannten Verbindungen hergestellt werden. So sind insbesondere 4-(Indol-3-yl)-buttersäure und 4-(Indol-3-yl)-4-oxo-buttersäure bekannt. Die übrigen Carbonsäuren der allgemeinen Formeln Ind–$(CH_2)_3$–COOH bzw. Ind–CO–$CH_2CH_2$–COOH sind analog aus den entsprechenden Indolen erhältlich. Primäre Alkohole der Formel II, worin die Gruppe A–$X^1$ eine –$(CH_2)_4$–OH– oder eine –CO$(CH_2)_3$–OH-Gruppe bedeutet, z.B. 4-(Indol-3-yl)-butanol, sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechende Halogenide der Formel II, worin A–$X^1$ = –$(CH_2)_4$–Cl oder –$(CH_2)_4$–Br, z.B. 3-(4-Chlorbutyl)-indol oder 3-(4-Brombutyl)-indol. Die Jodverbindungen, z.B. 3-(4-Jodbutyl)-indol, erhält man z.B. durch Einwirkung von Kaliumjodid auf die zugehörigen p-Toluolsulfonsäureester. Die entsprechenden Sulfonyloxyverbindungen sind erhältlich aus den Alkoholen Ind–A–OH durch Umsetzung mit den entsprechenden Sulfonsäurechloriden. Die Amine Ind–A–$NH_2$ sind z.B. aus den Halogeniden mit Phthalimidkalium oder durch Reduktion der entsprechenden Nitrile erhältlich.

Die Piperidinderivate IIIa sind grösstenteils bekannt (vgl. DE-OS 2 060 816) und z.B. erhältlich durch Umsetzung von 4-Piperidon mit metallorganischen Verbindungen der Formel M–Ar, anschliessende Hydrolyse zu den entsprechenden 4-Ar-4-hydroxy-piperidinen sowie gewünschtenfalls nachfolgende Dehydratisierung zu 4-Ar-3,4-dehydro-piperidinen und Hydrierung zu 4-Ar-piperidinen. Verbindungen der Formel III ($X^2$ und $X^3$ = jeweils X) sind z.B. herstellbar durch Reduktion von 3-Ar-glutarsäureestern zu 3-Ar-1,5-pentandiolen und gegebenenfalls anschliessende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Äthanol, Isopropanol, n-Butanol; Äther wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triäthylamin, Dimethylanilin, Pyridin oder Chinolin oder eines Überschusses der Aminkomponente Ind–A–$NH_2$ bzw. des Piperidinderivates der Formel IIIa kann günstig sein. Die Herstellung von Säurepiperididen der Formel I (A = –$(CH_2)_3$–CO–) gelingt z.B aus den freien Carbonsäuren der Formel II (A–$X^1$ = –$(CH_2)_3$–COOH) und Piperidinderivaten der Formel IIIa in Gegenwart eines Dehydratisierungsmittels, z.B. Carbonyldiimidazol oder Dicyclohexylcarbodiimid in einem der angegebenen inerten Lösungsmittel, bevorzugt THF.

Die Reaktionszeit liegt je nach den angewendeten Bedingungen zwischen einigen Minuten und 14 Tagen, die Reaktionstemperatur zwischen etwa 0 und 150°C, normalerweise zwischen 20 und 130°.

Gemäss einer Variante der vorstehenden Methode kann man eine Verbindung, die der Formel II entspricht, aber anstelle einer –CHX-Gruppe eine Carbonylgruppe besitzt, insbesondere einen Aldehyd der Formel Ind–$(CH_2)_3$–CHO oder Ind–CO–$(CH_2)_2$–CHO, mit einem Amin der Formel IIIa unter den Bedingungen einer katalytischen Hydrierung umsetzen. Die Reaktionsbedingungen entsprechen den aus der Literatur für reduktive Alkylierungen bekannten; als Zwischenprodukte entstehen vermutlich die entsprechenden Aldehyd-ammoniake. Die genannten Carbonylverbindungen, insbesondere die genannten Aldehyde, sind z.B. durch Oxydation der entsprechenden primären Alkohole der Formel II oder durch Hydrierung der entsprechenden Säurechloride in Gegenwart von Pd/$BaSO_4$-Katalysatoren zugänglich.

Die Cyclisierung von Isonitrilen der Formel IV mit Hilfe von Lithium-2,2,6,6-tetramethylpiperidid wird zweckmässig bei Temperaturen zwischen –100 und 0°, vorzugsweise zwischen –80 und –70°, in einem inerten Lösungsmittel, z.B. einem Glykoläther wie Diglyme, durchgeführt. Die Ausgangsstoffe der Formel IV sind z.B. in situ aus o-Tolylisonitril (das im Benzolring ein- bis dreifach durch Alkyl, O-Alkyl, S-Alkyl, OH, F, Cl, Br, $CF_3$ und/oder CN substituiert sein kann) und einem Halogenid der Formel VI

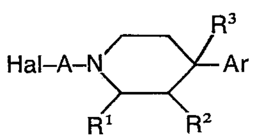

VI

worin

Hal Cl oder Br bedeutet und

$R^1$, $R^2$, $R^3$, A und Ar die angegebene Bedeutung haben,

erhältlich (vgl. J. Am. Chem. Soc. 99, (1977) 3532).

Eine Reduktion der Pyridiniumsalze der Formel V (worin An vorzugsweise Cl oder Br bedeutet) kann z.B. mit $NaBH_4$ in Wasser, einem Alkohol wie Methanol oder Äthanol oder in Gemischen dieser Lösungsmittel, falls erwünscht unter Zusatz einer Base wie NaOH, bei Temperaturen zwischen etwa 0 und 80° zu den entsprechenden 3,4-Dehydropiperidinderivaten (I, $R^2$ und $R^3$ = C–C-Bindung) führen.

Eine katalytische Hydrierung von V liefert dagegen in der Regel Piperidinderivate der Formel I ($R^2$ = $R^3$ = H). Für katalytische Hydrierung sind als Katalysatoren beispielsweise Edelmetall-, Nickel- und Kobaltkatalysatoren geeignet. Die Edelmetallkatalysatoren können auf Trägern (z.B. Platin oder Palladium auf Kohle, Palladium auf Calciumcarbonat oder Strontiumcarbonat), als Oxidkatalysatoren (z.B. Platinoxid), oder als feinteilige Metallkatalysatoren vorliegen. Nickel- und Kobaltkatalysatoren werden zweckmässig als Raney-Metalle, Nickel auch auf Kieselgur oder Bimsstein als Träger eingesetzt. Die Hydrierung kann bei Raumtemperatur und Normaldruck oder auch bei erhöhter Temperatur und/oder erhöhtem Druck durchgeführt werden. Vorzugsweise arbeitet man bei Drücken zwischen 1 und 100 at und bei Temperaturen zwischen –80 und +150°, in erster Linie zwischen Raumtemperatur und +100°. Die Umsetzung wird zweckmässig im sauren, neutralen oder basischen Bereich und in Gegenwart eines Lösungsmittels, wie Wasser, Methanol, Äthanol, Isopropanol, n-Butanol, Äthylacetat, Dioxan, Essigsäure oder THF, durchgeführt; man kann auch Gemische dieser Lösungsmittel untereinander anwenden.

Weiterhin kann man gegebenenfalls in einer Verbindung der Formel I eine oder beide CO-Gruppen zu $CH_2$-Gruppen reduzieren, beispielsweise mit Diboran oder mit einem komplexen Metallhydrid wie $LiAlH_4$ in einem Äther wie THF, und/oder eine Doppelbindung hydrieren, beispielsweise katalytisch nach einer der oben angegebenen Methoden.

Man kann ferner eine Verbindung der Formel I ($R^4$ = H) alkylieren, wobei man Verbindungen der Formel I ($R^4$ = Alkyl) erhält.

Für die Alkylierung eignen sich als Alkylierungsmittel beispielsweise Methylchlorid, -bromid, -jodid, -p-toluolsulfonat, Dimethylsulfat, Äthylchlorid, -bromid oder -jodid, n-Propylchlorid, -bromid oder -jodid, Isopropylchlorid, -bromid oder -jodid, n-Butylchlorid, -bromid oder -jodid, Isobutylchlorid, -bromid oder -jodid. Vor der Alkylierung werden die Verbindungen der Formel I zweckmässig in ihre Metallderivate umgewandelt, z.B. durch Umsetzung mit einem Alkoholat wie Natriumäthylat oder Kalium-tert.-butylat, einem Hydrid wie Natriumhydrid, einem Amid wie Natriumamid oder Lithium-diisopropylamid, einer metallorganischen Verbindung wie n-Butyl-Li oder einem Metall wie Natrium (z.B. in flüssigem Ammoniak). Diese Umwandlung geschieht zweckmässig in einem inerten Lösungsmittel, z.B. einem Alkohol wie Methanol, Äthanol oder tert.-Butanol, einem Äther wie Diäthyläther, einem Amid wie DMF oder einem Kohlenwasserstoff wie Benzol, ferner auch in Gemischen dieser Lösungsmittel. Die Alkylierung wird zweckmässig anschliessend in dem gleichen Reaktionsgemisch vorgenommen. Die Reaktionstemperaturen liegen in der Regel zwischen etwa –20 und +120°, vorzugsweise zwischen etwa 0 und 80°, die Reaktionszeiten vorzugsweise zwischen etwa 1 und 48 Stunden.

Äther der Formel I, in denen der Benzolring des Restes Ind ein- bis dreifach durch O-Alkyl substituiert ist, können nach Methoden, die aus der Literatur bekannt sind, gespalten werden, wobei die entsprechenden Hydroxyindolderivate entstehen. Zum Beispiel kann man die Äther spalten durch Behandeln mit HBr oder HJ in wässriger oder essigsaurer Lösung, durch Erhitzen mit Lewis-Säuren wie $AlCl_3$ oder Bortrihalogeniden oder durch Verschmelzen mit Pyridin- oder Anilin-hydrohalogeniden, vorzugsweise Pyridinhydrochlorid, bei etwa 150–250°.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Apfelsäure, Benzoesäure, Salicylsäure, 2-Phenylpropionsäure, Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsäure, Naphthalinmono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nichtchemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Säureadditionssalze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topikale Applikation eignen und mit den neuen Verbindungen nicht reagieren, beispielsweise Wasser, pflanzliche Öle, Benzylalkohole, Polyäthylenglykole, Gelatine, Kohlehydrate wie Lactose oder Stärke, Magnesiumstearat, Talk, Vaseline. Zur enteralen Applikation dienen insbesondere Tabletten, Dragées, Kapseln, Sirupe, Säfte, Tropfen oder Suppositorien, zur parenteralen Applikation Lösungen, vorzugsweise ölige oder wässrige Lösungen, ferner Suspensionen, Emulsionen oder Implantate, für die topikale Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Gegenstand der Erfindung ist ferner die Verwendung der Verbindungen der Formel I und ihrer physiologisch unbedenklichen Säureadditionssalze bei der therapeutischen Behandlung des menschlichen oder tierischen Körpers und bei der Bekämpfung von Krankheiten, insbesondere von Parkinsonismus, von extrapyramidalen Störungen bei der Neuroleptikatherapie, von Depressionen und von Nebenwirkungen bei der Behandlung der Hypertonie (z. B. mit $\alpha$-Methyldopa). Ferner können die Verbindungen in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z. B Migräne), insbesondere in der Geriatrie ähnlich wie gewisse Ergot-Alkaloide.

Dabei werden die erfindungsgemässen Substanzen in der Regel in Analogie zu bekannten, im Handel befindlichen Präparaten (z. B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jede der in den folgenden Beispielen genannten Verbindungen der Formel I ist zur Herstellung von pharmazeutischen Zubereitungen besonders geeignet.

In den nachfolgenden Beispielen bedeutet «übliche Aufarbeitung»:

Man gibt, falls erforderlich, Wasser hinzu, extrahiert mit einem organischen Lösungsmittel wie Benzol, Chloroform oder Dichlormethan, trennt ab, trocknet die organische Phase über Natriumsulfat, filtriert, dampft ein und reinigt durch Chromatographie und/oder Kristallisation. Temperaturen sind in Celsiusgraden angegeben.

Beispiel 1

a) Man rührt eine Lösung von 2,08 g 3-(4-Chlorbutyl)-indol (oder 2,52 g 3-(4-Brombutyl)-indol) und 1,6 g 4-Phenyl-3,4-dehydro-piperidin in 10 ml Acetonitril 12 Stunden bei 20°, arbeitet wie üblich auf und erhält

3-[4-(4-Phenyl-3,4-dehydro-1-piperidyl)-

butyl-indol-hydrochlorid, F 206–208°.

b) Man löst 3,3 g 3-[4-(4-Phenyl-3,4-dehydro-1-piperidyl)-butyl]-indol in 25 ml DMF, versetzt unter Rühren und Kühlen mit 0,24 g NaH, rührt noch 30 Minuten und gibt eine Lösung von 2 g Methyljodid in 10 ml DMF hinzu. Das Gemisch wird 16 Stunden bei 25° gerührt. Nach üblicher Aufarbeitung erhält man
1-Methyl-3-[4-(4-phenyl-3,4-dehydro-1-piperidyl)-butyl]-indol,
Rf 0,65 (Toluol/Äthylacetat 95:5, an Kieselgel).

Beispiele 2 bis 58

Analog Beispiel 1 erhält man aus den entsprechenden 3-(Chloralkyl)- bzw. 3-(Bromalkyl)-indolen und den entsprechenden Piperidinderivaten:

2. 1-Methyl-3-[4-(4-phenyl-3,4-dehydro-1-piperidyl)-butyl]-indol, Rf 0,65 (Toluol/Äthylacetat 95:5).
3. 1-Äthyl-3-[4-(4-phenyl-3,4-dehydro-1-piperidyl)-butyl]-indol.
4. 1-n-Butyl-3-[4-(4-phenyl-3,4-dehydro-1-piperidyl)-butyl]-indol.
5. 1-Phenyl-3-[4-(4-phenyl-3,4-dehydro-1-piperidyl)-butyl]-indol, Hydrochlorid, F. 221°.
6. 2-Methyl-3-[4-(4-phenyl-3,4-dehydro-1-piperidyl)-butyl]-indol, Hydrochlorid, F. 216–218°.
7. 2-Äthyl-3-[4(4-phenyl-3,4-dehydro-1-piperidyl)-butyl]-indol.
8. 2-n-Butyl-3-[4-(4-phenyl-3,4-dehydro-1-piperidyl)-butyl]-indol.
9. 2-Phenyl-3-[4-(4-phenyl-3,4-dehydro-1-piperidyl)-butyl]-indol, Hydrochlorid, F. 193–195°.
10. 1,2-Dimethyl-3-[4-(4-phenyl-3,4-dehydro-1-piperidyl)-butyl]-indol.
11. 3-[4-(4-Phenyl-3,4-dehydro-1-piperidyl)-butyl]-5-methyl-indol.
12. 3-[4-(4-Phenyl-3,4-dehydro-1-piperidyl)-butyl]-5-äthyl-indol, F. 152–154°.
13. 3-[4-(4-Phenyl-3,4-dehydro-1-piperidyl)-butyl]-5-methoxy-indol, Hydrochlorid, F. 181°.
14. 3-[4-(4-Phenyl-3,4-dehydro-1-piperidyl)-butyl]-5-fluor-indol, Hydrochlorid, F. 229°.
15. 3-[4-(4-Phenyl-3,4-dehydro-1-piperidyl)-butyl]-5-chlor-indol, Hydrochlorid, F. 226–228°.
16. 3-[4-(4-Phenyl-3,4-dehydro-1-piperidyl)-butyl[5-brom-indol.
17. 3-[4-(4-Phenyl-3,4-dehydro-1-piperidyl)-butyl]-5-trifluormethyl-indol.
18. 3-[4-(4-Phenyl-3,4-dehydro-1-piperidyl)-butyl]-5-cyan-indol.
19. 3-[4-(4-Phenyl-3,4-dehydro-1-piperidyl)-butyl-6-methoxy-indol.
20. 3-[4-(4-Phenyl-3,4-dehydro-1-piperidyl)-butyl]-7-methyl-indol, Hydrochlorid, F. 206–208°.
21. 3-[4-(4-Phenyl-3,4-dehydro-1-piperidyl)-butyl]-7-methylthio-indol.
22. 3-[4-(4-Phenyl-3,4-dehydro-1-piperidyl)-butyl]-7-trifluormethyl-indol.
23. 2,5-Dimethyl-3-[4-(4-phenyl-3,4-dehydro-1-piperidyl)-butyl]-indol.
24. 2-Methyl-3-[4-(4-phenyl-3,4-dehydro-1-piperidyl)-butyl]-5-methoxy-indol, F. 142–144°.
25. 2-Methyl-3-[4-(4-phenyl-3,4-dehydro-1-piperidyl)-butyl]-5-chlor-indol, Hydrochlorid, F. 222–224°.
26. 3-[4-(4-Phenyl-3,4-dehydro-1-piperidyl)-butyl]-4,7-dimethyl-indol.
27. 3-[4-(4-Phenyl-3,4-dehydro-1-piperidyl)-butyl]-4,5,6-trimethoxy-indol.
28. 3-[4-(4-Phenyl-3,4-dehydro-1-piperidyl)-butyl]-4-trifluormethyl-7-chlor-indol.
29. 3-[4-(4-Phenyl-3,4-dehydro-1-piperidyl)-butyl]-5-methyl-7-chlor-indol.
30. 3-[4-(4-Phenyl-3,4-dehydro-1-piperidyl)-butyl]-5,6-dimethoxy-indol.
31. 3-[4-(4-Phenyl-3,4-dehydro-1-piperidyl)-butyl]-5,7-dimethoxy-indol.
32. 3-[4-(4-Phenyl-3,4-dehydro-1-piperidyl)-butyl]-5-fluor-7-methyl-indol.
33. 3-[4-(4-Phenyl-3,4-dehydro-1-piperidyl)-butyl]-5,7-dichlor-indol.
34. 3-[4-(4-Phenyl-3,4-dehydro-1-piperidyl)-butyl]-5-chlor-7-methyl-indol.
35. 3-[4-(4-Phenyl-3,4-dehydro-1-piperidyl)-butyl]-5,6,7-trichlor-indol.
36. 3-[4-(2-Methyl-4-phenyl-3,4-dehydro-1-piperidyl)-butyl]-indol, Hydrochlorid, F. 218–220°.
37. 3-[4-(4-Phenyl-1-piperidyl)-butyl]-indol, Hydrochlorid, F. 213–215°.
38. 1-Methyl-3-[4-(4-phenyl-1-piperidyl)-butyl]-indol.
39. 1-Phenyl-3-[4-(4-phenyl-1-piperidyl)-butyl]-indol.
40. 2-Methyl-3-[4-(4-phenyl-1-piperidyl)-butyl]-indol.
41. 3-[4-(4-p-Tolyl-3,4-dehydro-1-piperidyl)-butyl]-indol.
42. 3-[4-(4-p-Fluorphenyl-3,4-dehydro-1-piperidyl)-butyl]-indol.
43. 3-[4-(4-o-Chlorphenyl-3,4-dehydro-1-piperidyl)-butyl]-indol.
44. 3-[4-(4-m-Chlorphenyl-3,4-dehydro-1-piperidyl)-butyl]-indol, Hydrochlorid, F. 260–262°.
45. 3-[4-(4-p-Chlorphenyl-3,4-dehydro-1-piperidyl)-butyl]-indol.
46. 3-[4-(4-p-Bromphenyl-3,4-dehydro-1-piperidyl)-butyl]-indol.
47. 3-[4-(4-p-Jodphenyl-3,4-dehydro-1-piperidyl)-butyl]-indol.
48. 3-[4-(4-m-Trifluormethylphenyl-3,4-dehydro-1-piperidyl)-butyl]-indol.
49. 1-Methyl-3-[4-(4-p-chlorphenyl-3,4-dehydro-1-piperidyl)-butyl]-indol.
50. 2-Methyl-3-[4-(4-p-chlorphenyl-3,4-dehydro-1-piperidyl)-butyl]-indol, Hydrochlorid, F. 243–245°.

51. 3-[4-(4-p-Tolyl-1-piperidyl)-butyl]-indol.
52. 3-[4-(4-p-Fluorphenyl-1-piperidyl)-butyl]-indol.
53. 3-[4-(4-o-Chlorphenyl-1-piperidyl)-butyl]-indol.
54. 3-[4-(4-m-Chlorphenyl-1-piperidyl)-butyl]-indol.
55. 3-[4-(4-p-Chlorphenyl-1-piperidyl)-butyl]-indol.
56. 3-[4-(4-p-Bromphenyl-1-piperidyl)-butyl]-indol.
57. 3-[4-(4-p-Jodphenyl-1-piperidyl)-butyl]-indol.
58. 3-[4-(4-m-Trifluormethylphenyl-1-piperidyl)-butyl]-indol.

Beispiel 59

Ein Gemisch von 4,43 g 3-(4-p-Toluolsulfonyloxy-butyl)-indol und 3,18 g 4-Phenyl-3,4-dehydro-piperidin wird auf 130° erhitzt. Nach Abklingen der exothermen Reaktion und Erkalten arbeitet man wie üblich auf und erhält
3-[4-(4-Phenyl-3,4-dehydro-1-piperidyl)-butyl]-indol,
Hydrochlorid, F. 206–208°.

Beispiel 60

Man kocht 2,99 g 3-(4-Jodbutyl)-indol, 1,59 g 4-Phenyl-3,4-dehydro-piperidin und 1,5 g wasserfreies Kaliumcarbonat in 25 ml n-Butanol 2 Stunden unter Rühren, lässt erkalten, arbeitet wie üblich auf und erhält
3-[4-(4-Phenyl-3,4-dehydro-1-piperidyl)-butyl]-indol,
Hydrochlorid, F. 206–208°.

Beispiel 61

a) Zu einer Lösung von 2,03 g 4-(3-Indolyl)-buttersäure in 10 ml THF gibt man 1,62 g Carbonyldiimidazol, rührt eine Stunde bei 20° und fügt dann eine Lösung von 1,59 g 4-Phenyl-3,4-dehydropiperidin in 50 ml THF hinzu. Man rührt 1,5 Stunden bei 20°, arbeitet wie üblich auf und erhält
3-[4-Oxo-4-(4-phenyl-3,4-dehydro-1-piperidyl)-butyl]-indol,
F. 129–131°.

b) Zu einer Suspension von 0,38 g $LiAlH_4$ in 10 ml THF tropft man eine Lösung von 3,44 g
3-[4-Oxo-4-(4-phenyl-3,4-dehydro-1-piperidyl)-butyl]-indol
in 10 ml THF unter Rühren. Nach Abklingen der Reaktion gibt man 5 ml Äthylacetat hinzu, arbeitet wie üblich auf und erhält
3-[4-(4-Phenyl-3,4-dehydro-1-piperidyl)-butyl]-indol,
Hydrochlorid, F. 206–208°.

Beispiele 62 bis 170

Analog Beispiel 61a) erhält man aus den entsprechenden 3-Indolyl-alkansäuren und den entsprechenden Aminen:

62. 1-Methyl-3-[4-oxo-4-(4-phenyl-3,4-dehydro-1-piperidyl)-butyl]-indol.
63. 1-Äthyl-3-[4-oxo-4-(4-phenyl-3,4-dehydro-1-piperidyl)-butyl]-indol.
64. 1-n-Butyl-3-[4-oxo-4-(4-phenyl-3,4-dehydro-1-piperidyl)-butyl]-indol.
65. 2-Methyl-3-[4-oxo-4-(4-phenyl-3,4-dehydro-1-piperidyl)-butyl]-indol.
66. 2-Äthyl-3-[4-oxo-4-(4-phenyl-3,4-dehydro-1-piperidyl)-butyl]-indol.
67. 2-n-Butyl-3-[4-oxo-4-(4-phenyl-3,4-dehydro-1-piperidyl)-butyl]-indol.
68. 2-Phenyl-3-[4-oxo-4-(4-phenyl-3,4-dehydro-1-piperidyl)-butyl]-indol.
69. 1,2-Dimethyl-3-[4-oxo-4-(4-phenyl-3,4-dehydro-1-piperidyl)-butyl]-indol.
70. 3-[4-Oxo-4-(4-phenyl-3,4-dehydro-3,4-1-piperidyl)-butyl]-5-methyl-indol.
71. 3-[4-Oxo-4-(4-phenyl-3,4-dehydro-1-piperidyl)-butyl]-5-äthyl-indol.
72. 3-[4-Oxo-4-(4-phenyl-3,4-dehydro-1-piperidyl)-butyl-]-5-methoxy-indol.
73. 3-[4-Oxo-4-(4-phenyl-3,4-dehydro-1-piperidyl)-butyl]-5-fluor-indol, F. 131°.
74. 3-[4-Oxo-4-(4-phenyl-3,4-dehydro-1-piperidyl)-butyl]-5-chlor-indol.
75. 3-[4-Oxo-4-(4-phenyl-3,4-dehydro-1-piperidyl)-butyl]-5-brom-indol.
76. 3-[4-Oxo-4-(4-phenyl-3,4-dehydro-1-piperidyl)-butyl]-5-trifluormethyl-indol.
77. 3-[4-Oxo-4-(4-phenyl-3,4-dehydro-1-piperidyl)-butyl]-5-cyan-indol.
78. 3-[4-Oxo-4-(4-phenyl-3,4-dehydro-1-piperidyl)-butyl]-6-methoxy-indol.
79. 3-[4-Oxo-4-(4-phenyl-3,4-dehydro-1-piperidyl)-butyl]-7-methyl-indol.
80. 3-[4-Oxo-4-(4-phenyl-3,4-dehydro-1-piperidyl)-butyl]-7-methylthio-indol.
81. 3-[4-Oxo-4-(4-phenyl-3,4-dehydro-1-piperidyl)-butyl]-7-trifluormethyl-indol.
82. 2,5-Dimethyl-3-[4-oxo-4-(4-phenyl-3,4-dehydro-1-piperidyl)-butyl]-indol.
83. 2-Methyl-3-[4-oxo-4-(4-phenyl-3,4-dehydro-1-piperidyl)-butyl]-5-methoxy-indol.
84. 2-Methyl-3-[4-oxo-4-(4-phenyl-3,4-dehydro-1-piperidyl)-butyl]-5-chlor-indol.
85. 3-[4-Oxo-4-(4-phenyl-3,4-dehydro-1-piperidyl)-butyl]-4,7-dimethyl-indol.
86. 3-[4-Oxo-4-(4-phenyl-3,4-dehydro-1-piperidyl)-butyl]4,5,6-trimethoxy-indol.
87. 3-[4-Oxo-4-(4-phenyl-3,4-dehydro-1-piperidyl)-butyl]-4-trifluormethyl-7-chlor-indol.
88. 3-[4-Oxo-4-(4-phenyl-3,4-dehydro-1-piperidyl)-butyl]-5-methyl-7-chlor-indol.
89. 3-[4-Oxo-4-(4-phenyl-3,4-dehydro-1-piperidyl)-butyl]-5,6-dimethoxy-indol.
90. 3-[4-Oxo-4-(4-phenyl-3,4-dehydro-1-piperidyl)-butyl]-5,7-dimethoxy-indol.
91. 3-[4-Oxo-4-(4-phenyl-3,4-dehydro-1-piperidyl)-butyl]-5-fluor-7-methyl-indol.
92. 3-[4-Oxo-4-(4-phenyl-3,4-dehydro-1-piperidyl)-butyl]-5,7-dichlor-indol.
93. 3-[4-Oxo-4-(4-phenyl-3,4-dehydro-1-piperidyl)-butyl]-5-chlor-7-methyl-indol.
94. 3-[4-Oxo-4-(4-phenyl-3,4-dehydro-1-piperidyl)-butyl]-5,6,7-trichlor-indol.

95. 3-[4-Oxo-4-(2-methyl-4-phenyl-3,4-dehydro-1-piperidyl)-butyl]-indol.
96. 3-[4-Oxo-4-(4-phenyl-1-piperidyl)-butyl]-indol.
97. 1-Methyl-3-[4-oxo-4-(4-phenyl-1-piperidyl)-butyl]-indol.
98. 3-[4-Oxo-4-(4-tolyl-3,4-dehydro-1-piperidyl)-butyl]-indol,
99. 3-[4-Oxo-4-(4-p-fluorphenyl-3,4-dehydro-1-piperidyl)-butyl]-indol.
100. 3-[4-Oxo-4-(4-o-chlorphenyl-3,4-dehydro-1-piperidyl)-butyl]-indol.
101. 3-[4-Oxo-4-(4-m-chlorphenyl-3,4-dehydro-1-piperidyl)-butyl]-indol.
102. 3-[4-Oxo-4-(4-p-chlorphenyl-3,4-dehydro-1-piperidyl)-butyl]-indol.
103. 3-[4-Oxo-4-(4-p-bromphenyl-3,4-dehydro-1-piperidyl)-butyl]-indol.
104. 3-[4-Oxo-4-(4-p-jodphenyl-3,4-dehydro-1-piperidyl)-butyl]-indol.
105. 3-[4-Oxo-4-(4-m-trifluormethylphenyl-3,4-dehydro-1-piperidyl)-butyl]-indol.
106. 1-Methyl-3-[4-oxo-4-(4-p-chlorphenyl-3,4-dehydro-1-piperidyl)-butyl]-indol.
107. 2-Methyl-3-[4-oxo-4-(4-p-chlorphenyl-3,4-dehydro-1-piperidyl)-butyl]-indol.
108. 3-[4-Oxo-4-(4-p-tolyl-1-piperidyl)-butyl]-indol.
109. 3-[4-Oxo-4-(4-p-fluorphenyl-1-piperidyl)-butyl]-indol.
110. 3-[4-Oxo-4-(4-o-chlorphenyl-1-piperidyl)-butyl]-indol.
111. 3-[4-Oxo-4-(4-m-chlorphenyl-1-piperidyl)-butyl]-indol.
112. 3-[4-Oxo-4-(4-p-chlorphenyl-1-piperidyl)-butyl]-indol.
113. 3-[4-Oxo-4-(4-p-bromphenyl-1-piperidyl)-butyl]-indol.
114. 3-[4-Oxo-4-(4-p-jodphenyl-1-piperidyl)-butyl]-indol.
115. 3-[4-Oxo-4-(4-m-trifluormethylphenyl-1-piperidyl)-butyl]-indol.
116. 3-[1,4-Dioxo-4-(4-phenyl-3,4-dehydro-1-piperidyl)-butyl]-indol.
117. 1-Methyl-3-[1,4-dioxo-4-(4-phenyl-3,4-dehydro-1-piperidyl)-butyl]-indol.
118. 1-Äthyl-3-[1,4-dioxo-4-(4-phenyl-3,4-dehydro-1-piperidyl)-butyl]-indol.
119. 1-n-Butyl-3-[1,4-dioxo-4-(4-phenyl-3,4-dehydro-1-piperidyl)-butyl]-indol.
120. 2-Methyl-3-[1,4-dioxo-4-(4-phenyl-3,4-dehydro-1-piperidyl)-butyl]-indol.
121. 2-Äthyl-3-[1,4-dioxo-4-(4-phenyl-3,4-dehydro-1-piperidyl)-butyl]-indol.
122. 2-n-Butyl-3-[1,4-dioxo-4-(4-phenyl-3,4-dehydro-1-piperidyl)-butyl]-indol.
123. 2-Phenyl-3-[1,4-dioxo-4-(4-phenyl-3,4-dehydro-1-piperidyl)-butyl]-indol, F. 152–154°.
124. 1,2-Dimethyl-3-[1,4-dioxo-4-(4-phenyl-3,4-dehydro-1-piperidyl)-butyl]-indol.
125. 3-[1,4-Dioxo-4-(4-phenyl-3,4-dehydro-1-piperidyl)-butyl]-5-methyl-indol.
126. 3-[1,4-Dioxo-4-(4-phenyl-3,4-dehydro-1-piperidyl)-butyl]-5-äthyl-indol.
127. 3-[1,4-Dioxo-4-(4-phenyl-3,4-dehydro-1-piperidyl)-butyl]-5-methoxy-indol.
128. 3-[1,4-Dioxo-4-(4-phenyl-3,4-dehydro-1-piperidyl)-butyl]-5-fluor-indol.
129. 3-[1,4-Dioxo-4-(4-phenyl-3,4-dehydro-1-piperidyl)-butyl]-5-chlor-indol, F. 200–202°.
130. 3-[1,4-Dioxo-4-(4-phenyl-3,4-dehydro-1-piperidyl)-butyl]-5-brom-indol.
131. 3-[1,4-Dioxo-4-(4-phenyl-3,4-dehydro-1-piperidyl)-butyl]-5-trifluormethyl-indol.
132. 3-[1,4-Dioxo-4-(4-phenyl-3,4-dehydro-1-piperidyl)-butyl]-5-cyan-indol, F. 220–222°.
133. 3-[1,4-Dioxo-4-(4-phenyl-3,4-dehydro-1-piperidyl)-butyl]-6-methoxy-indol.
134. 3-[1,4-Dioxo-4-(4-phenyl-3,4-dehydro-1-piperidyl)-butyl]-7-methyl-indol.
135. 3-[1,4-Dioxo-4-(4-phenyl-3,4-dehydro-1-piperidyl)-butyl]-7-methylthio-indol.
136. 3-[1,4-Dioxo-4-(4-phenyl-3,4-dehydro-1-piperidyl)-butyl]-7-trifluor-indol.
137. 2,5-Dimethyl-3-[1,4-dioxo-4-(4-phenyl-3,4-dehydro-1-piperidyl)-butyl]-indol, F. 201–203°.
138. 2-Methyl-3-[1,4-dioxo-4-(4-phenyl-3,4-dehydro-1-piperidyl)-butyl]-5-methoxy-indol, F. 185–187°.
139. 2-Methyl-3-[1,4-dioxo-4-(4-phenyl-3,4-dehydro-1-piperidyl)-butyl]-5-chlor-indol.
140. 3-[1,4-Dioxo-4-(4-phenyl-3,4-dehydro-1-piperidyl)-butyl]-4,7-dimethyl-indol.
141. 3-[1,4-Dioxo-4-(4-phenyl-3,4-dehydro-1-piperidyl)-butyl]-4,5,6-trimethoxy-indol.
142. 3-[1,4-Dioxo-4-(4-phenyl-3,4-dehydro-1-piperidyl)-butyl]-4-trifluormethyl-7-chlor-indol.
143. 3-[1,4-Dioxo-4-(4-phenyl-3,4-dehydro-1-piperidyl)-butyl]-5-methyl-7-chlor-indol.
144. 3-[1,4-Dioxo-4-(4-phenyl-3,4-dehydro-1-piperidyl)-butyl]-5,6-dimethoxy-indol.
145. 3-[1,4-Dioxo-4-(4-phenyl-3,4-dehydro-1-piperidyl)-butyl]-5,7-dimethoxy-indol.
146. 3-[1,4-Dioxo-4-(4-phenyl-3,4-dehydro-1-piperidyl)-butyl]-5-fluor-7-methyl-indol.
147. 3-[1,4-Dioxo-4-(4-phenyl-3,4-dehydro-1-piperidyl)-butyl]-5,7-dichlor-indol.
148. 3-[1,4-Dioxo-4-(4-phenyl-3,4-dehydro-1-piperidyl)-butyl]-5-chlor-7-methyl-indol.
149. 3-[1,4-Dioxo-4-(4-phenyl-3,4-dehydro-1-piperidyl)-butyl]-5,6,7-trichlor-indol.
150. 3-[1,4-Dioxo-4-(2-methyl-4-phenyl-3,4-dehydro-1-piperidyl)-butyl]-indol.
151. 3-[1,4-Dioxo-4-(4-phenyl-1-piperidyl)-butyl]-indol.
152. 1-Methyl-3-[1,4-dioxo-4-(4-phenyl-1-piperidyl)-butyl]-indol.
153. 3-[1,4-Dioxo-4-(4-p-tolyl-3,4-dehydro-1-piperidyl)-butyl]-indol.
154. 3-[1,4-Dioxo-4-(4-p-fluorphenyl-3,4-dehydro-1-piperidyl)-butyl]-indol.
155. 3-[1,4-Dioxo-4-(4-o-chlorphenyl-3,4-dehydro-1-piperidyl)-butyl]-indol.

156. 3-[1,4-Dioxo-4-(4-m-chlorphenyl-3,4-dehydro-1-piperidyl)-butyl]-indol.
157. 3-[1,4-Dioxo-4-(4-p-chlorphenyl-3,4-dehydro-1-piperidyl)-butyl]-indol.
158. 3-[1,4-Dioxo-4-(4-p-bromphenyl-3,4-dehydro-1-piperidyl)-butyl]-indol.
159. 3-[1,4-Dioxo-4-(4-p-jodphenyl-3,4-dehydro-1-piperidyl)-butyl]-indol.
160. 3-[1,4-Dioxo-4-(4-m-trifluormethylphenyl-3,4-dehydro-1-piperidyl)-butyl]-indol.
161. 1-Methyl-3-[1,4-dioxo-4-(4-p-chlorphenyl-3,4-dehydro-1-piperidyl)-butyl]-indol.
162. 2-Methyl-3-[1,4-dioxo-4-(4-p-chlorphenyl-3,4-dehydro-1-piperidyl)-butyl]-indol, F. 227–229°.
163. 3-[1,4-Dioxo-4-(4-p-tolyl-1-piperidyl)-butyl]-indol.
164. 3-[1,4-Dioxo-4-(4-p-fluorphenyl-1-piperidyl)-butyl]-indol.
165. 3-[1,4-Dioxo-4-(4-o-chlorphenyl-1-piperidyl)-butyl]-indol.
166. 3-[1,4-Dioxo-4-(4-m-chlorphenyl-1-piperidyl)-butyl]-indol.
167. 3-[1,4-Dioxo-4-(4-p-chlorphenyl-1-piperidyl)-butyl]-indol.
168. 3-[1,4-Dioxo-4-(4-p-bromphenyl-1-piperidyl)-butyl]-indol.
169. 3-[1,4-Dioxo-4-(4-p-jodphenyl-1-piperidyl)-butyl]-indol.
170. 3-[1,4-Dioxo-4-(4-m-trifluormethylphenyl-1-piperidyl)-butyl]-indol.

Beispiel 171

Ein Gemisch von 1,88 g 3-(4-Aminobutyl)-indol [erhältlich durch Reaktion von 3-(4-Brombutyl)-indol mit Phthalimidkalium und anschliessende Hydrolyse] und 2,15 g 1,5-Dichlor-3-phenyl-2-penten (erhältlich durch Reduktion von 3-Phenyl-2-penten-1,5-disäurediäthylester mit $LiAlH_4$ und anschliessende Reaktion mit $SOCl_2$) in 40 ml Aceton und 40 ml Wasser wird 24 Stunden gekocht und wie üblich aufgearbeitet. Man erhält 3-[4-(4-Phenyl-3,4-dehydro-1-piperidyl)-butyl]-indol, Hydrochlorid, F. 206–208°.

Beispiel 172

Zu einer Lösung von 1,17 g o-Tolylisonitril in 10 ml Diglyme tropft man bei –78° eine Lösung von 2,14 g Lithiumdiisopropylamid in 10 ml Diglyme. Die erhaltene Lösung wird bei –78° mit 5,88 g 4-(4-Phenyl-3,4-dehydro-1-piperidyl)-butylbromid versetzt. Nach üblicher Aufarbeitung erhält man o-[4-(4-Phenyl-3,4-dehydro-1-piperidyl)-butyl]-phenyl-isonitril, das roh in 10 ml Diglyme aufgenommen und bei –78° mit einer Lösung von 2,94 g Lithium-2,2,6,6-tetramethyl-piperidid versetzt. Man lässt die Temperatur auf 20° kommen, arbeitet wie üblich auf und erhält 3-[4-(4-Phenyl-3,4-dehydro-1-piperidyl)-butyl]-indol, Hydrochlorid, F. 206–208°.

Beispiel 173

a) Zu einer Lösung von 4.07 g 1-[4-(3-Indolyl)-butyl]-4-phenylpyridinium-bromid [erhältlich aus 3-(4-Brombutyl)-indol und 4-Phenyl-pyridin] in 50 ml Ina NaOH gibt man unter Rühren 1 g $NaBH_4$ in 20 ml Wasser und rührt danach noch 3 Stunden bei 60°. Nach üblicher Aufarbeitung erhält man 3-[4-(4-Phenyl-3,4-dehydro-1-piperidyl)-butyl]-indol, Hydrochlorid, F. 206–208°.

b) Man löst 1 g 3-[4-(4-Phenyl-3,4-dehydro-1-piperidyl)-butyl]-indol in 25 ml Dioxan, hydriert an 0,2 g 5%iger Pd-Kohle bei 40° und Normaldruck bis zum Stillstand, filtriert, dampft ein und erhält nach üblicher Aufarbeitung 3-[4-(4-Phenyl-1-piperidyl)-butyl]-indol, Hydrochlorid, F. 213–215.

Beispiel 174

Man löst 1 g 1-[4-(3-Indolyl)-butyl]-4-phenylpyridiniumchlorid in 25 ml Methanol, hydriert an 0,1 g Platin bei 25° und Normaldruck bis zum Stillstand, filtriert, dampft ein und erhält nach üblicher Aufarbeitung 3-[4-(4-Phenyl-1-piperidyl)-butyl]-indol, Hydrochlorid, F. 213–215°.

Beispiel 175

Ein Gemisch aus 3,97 g 3-[4-(4-Phenyl-3,4-dehydro-1-piperidyl)-butyl]-5-methoxyindol-hydrochlorid und 3,5 g Pyridinhydrochlorid wird 3 Stunden bei 160° gerührt. Nach üblicher Aufarbeitung erhält man 3-[4-(4-Phenyl-3,4-dehydro-1-piperidyl)-butyl]-5-hydroxy-indol, Hydrochlorid, F. 258°.

Beispiel 176

Analog Beispiel 175 erhält man aus der entsprechenden 6-Methoxy-Verbindung das 3-[4-(4-Phenyl-3,4-dehydro-1-piperidyl)-butyl]-6-hydroxy-indol.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg 3-[4-(4-Phenyl-3,4-dehydro-1-piperidyl)-butyl]-indol-hydrochlorid, 4 kg Lactose, 1,2 kg Kartoffelstärke, 0,2 kg Talk und 0,1 kg Magnesiumstearat wird in üblicher Weise zu Tabletten gepresst, derart, dass jede Tablette 10 mg Wirkstoff enthält.

Beispiel B: Dragées

Analog Beispiel A werden Tabletten gepresst, die anschliessend in üblicher Weise mit einem Überzug aus Saccharose, Kartoffelstärke, Talk, Tragant und Farbstoff überzogen werden.

Beispiel C: Kapseln

2 g
3-[4-(4-Phenyl-3,4-dehydro-1-piperidyl)-butyl]-indol-hydrochlorid
werden in üblicher Weise in Hartgelatinekapseln gefüllt, so dass jede Kapsel 20 mg des Wirkstoffs enthält.

Beispiel D: Ampullen

Eine Lösung von 1 kg
3-[4-(4-Phenyl-3,4-dehydro-1-piperidyl)-butyl]-indol-hydrochlorid
in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff. Analog sind Tabletten, Dragées, Kapseln und Ampullen erhältlich, die einen oder mehrere der übrigen Wirkstoffe der Formel I und/oder ihre physiologisch unbedenklichen Säureadditionssalze enthalten.

In der US-A 3 217 011, der US-A 3 238 215 und der DE-A 2 060 816 sind Verbindungen gleicher oder ähnlicher Wirkungsrichtung beschrieben, die den vorliegenden Verbindungen teilweise konstitutionell ähnlich sind, so insbesondere das 3-[4-(4-Benzyl-piperidyl)-butyl]-indol (vgl. US-A 3 238 215, Beispiel 123). Dieser Substanz gegenüber zeigen die vorliegenden Verbindungen jedoch bis zu etwa 150mal so starke Wirkungen im Drehverhaltens-Test an Hemiparkinson-Ratten (vgl. Ungerstedt et al., l.c.).

**Patentansprüche**

für die Vertragsstaaten: BE, CH, DE, FR, GB, IT, NL, SE

1. Indolalkylamine der allgemeinen Formel I

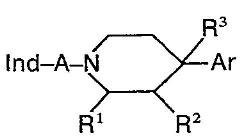

I

worin

Ind einen 1-$R^4$-2-$R^5$-Indol-3-ylrest, der im Benzolring ein- bis dreifach durch Alkyl, O-Alkyl, S-Alkyl, OH, F, Cl, Br, $CF_3$ und/oder CN substituiert sein kann,
A $-(CH_2)_4-$, $-(CH_2)_3-CO-$ oder $-CO-(CH_2)_2-CO-$,
$R^1$ H oder Methyl,
$R^2$ H oder zusammen mit $R^3$ eine C–C-Bindung,
$R^3$ H oder zusammen mit $R^2$ eine C–C-Bindung,
$R^4$ und $R^5$ jeweils H, Alkyl oder Phenyl und
Ar eine unsubstituierte oder ein- oder zweifach durch Alkyl, F, Cl, Br, J und/oder $CF_3$ substituierte Phenylgruppe
bedeuten und
worin die Alkylgruppen jeweils 1–4 C-Atome besitzen,
sowie deren physiologisch unbedenkliche Säureadditionssalze.

2. 3-4-(4-Phenyl-3,4-dehydro-1-piperidyl)-butyl]-indol.

3. Verfahren zur Herstellung von Indolalkylaminen der allgemeinen Formel I nach Patentanspruch 1 sowie von deren physiologisch unbedenklichen Säureadditionssalzen, dadurch gekennzeichnet, dass man eine Verbindung der allgemeinen Formel II

$$Ind-A-X^1 \qquad II$$

worin
$X^1$ X oder $NH_2$ und
X Cl, Br, J, OH oder eine reaktionsfähig funktionell abgewandelte OH-Gruppe bedeuten und
Ind und A die angegebenen Bedeutungen haben,
mit einer Verbindung der allgemeinen Formel III

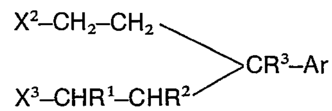

III

worin
$X^2$ und $X^3$ gleich oder verschieden sein können und falls $X^1 = NH_2$ ist, jeweils X, andernfalls zusammen NH bedeuten und
$R^1$, $R^2$, $R^3$ und AR die angegebenen Bedeutungen haben
umsetzt oder dass man ein Isonitril der allgemeinen Formel IV

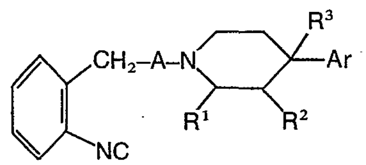

IV

worin
der Benzolring ein- bis dreifach durch Alkyl, O-Alkyl, S-Alkyl, OH, F, Cl, Br, $CF_3$ und/oder CN substituiert sein kann und
$R^1$, $R^2$, $R^3$, A und Ar die angegebenen Bedeutungen haben
mit Hilfe von Lithium-2,2,6,6-tetramethylpiperidid cyclisiert oder dass man ein Pyridiniumsalz der allgemeinen Formel V

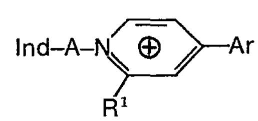

$An^{\ominus}$ V

worin
$An^{\ominus}$ ein Anion einer starken Säure bedeutet und
Ind, A, $R^1$ und Ar die angegebenen Bedeutungen haben
mit einem reduzierenden Mittel behandelt und/oder dass man gegebenenfalls in einer Verbindung der Formel I eine oder zwei CO-Gruppen zu $CH_2$-Gruppen reduziert und/oder eine Doppelbindung hydriert und/oder eine freie NH-Gruppe im Indolring alkyliert und/oder eine Alkoxygruppe unter Bildung einer Hydroxygruppe spaltet und/oder dass man eine Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, dass man eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit

einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze.

**Patentansprüche**
für den Vertragsstaat: AT

1. Verfahren zur Herstellung von Indolalkylaminen der allgemeinen Formel I

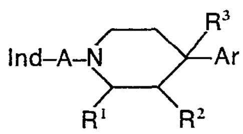 I

worin
Ind einen 1-$R^4$-2-$R^5$-Indol-3-ylrest, der im Benzolring ein- bis dreifach durch Alkyl, O-Alkyl, S-Alkyl, OH, F, Cl, Br, $CF_3$, und/oder CN substituiert sein kann,
A $-(CH_2)_4-$, $-(CH_2)_3-CO-$ oder $-CO-(CH_2)_2-CO-$,
$R^1$ H oder Methyl,
$R^2$ H oder Zusammen mit $R^3$ eine C–C-Bindung,
$R^3$ H oder zusammen mit $R^2$ eine C–C-Bindung,
$R^4$ und $R^5$ jeweils H, Alkyl oder Phenyl und
Ar eine unsubstituierte oder ein- oder zweifach durch Alkyl, F, Cl, Br, J und/oder $CF_3$ substituierte Phenylgruppe
bedeuten und
worin die Alkylgruppen jeweils 1–4 C-Atome besitzen,
sowie von deren physiologisch unbedenklichen Säureadditionssalzen, dadurch gekennzeichnet, dass man eine Verbindung der allgemeinen Formel II

Ind–A–$X^1$ II

worin
$X^1$ X oder $NH_2$ und
X Cl, Br, J, OH oder eine reaktionsfähig funktionell abgewandelte OH-Gruppe bedeuten und
Ind und A die angegebenen Bedeutungen haben, mit einer Verbindung der allgemeinen Formel III

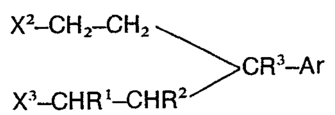 III

worin
$X^2$ und $X^3$ gleich oder verschieden sein können und falls $X^1$ = $NH_2$ ist, jeweils X, andernfalls zusammen NH bedeuten und
$R^1$, $R^2$, $R^3$ und Ar die angegebenen Bedeutungen haben
umsetzt oder dass man ein Isonitril der allgemeinen Formel IV

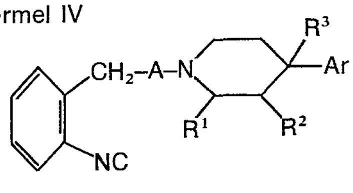 IV

worin
der Benzolring ein- bis dreifach durch Alkyl, O-Alkyl, S-Alkyl, OH, F, Cl, Br, $CF_3$ und/oder CN substituiert sein kann und
$R^1$, $R^2$, $R^3$, A und Ar die angegebenen Bedeutungen haben
mit Hilfe von Lithium-2,2,6,6-tetramethylpiperidid cyclisiert oder dass man ein Pyridiniumsalz der allgemeinen Formel V

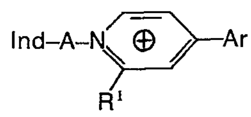 $An^{\ominus}$ V

worin
$An^{\ominus}$ ein Anion einer starken Säure bedeutet und Ind, A, $R^1$ und Ar die angegebenen Bedeutungen haben
mit einem reduzierenden Mittel behandelt und/oder dass man gegebenenfalls in einer Verbindung der Formel I eine oder zwei CO-Gruppen zu $CH_2$-Gruppen reduziert und/oder eine Doppelbindung hydriert und/oder eine freie NH-Gruppe im Indolring alkyliert und/oder eine Alkoxygruppe unter Bildung einer Hydroxygruppe spaltet und/oder dass man eine Base der Formel I durch Behandeln mit einer Säure in eines ihrer physiologisch unbedenklichen Säureadditionssalze umwandelt (Priorität: 16.3.1979).

2. Verfahren zur Herstellung von Indolalkylaminen der allgemeinen Formel I

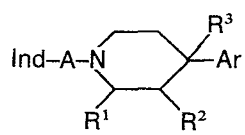 I

worin
Ind einen 1-$R^4$-2-$R^5$-Indol-3-ylrest, der im Benzolring ein- bis dreifach durch Alkyl, O-Alkyl, S-Alkyl, F, Cl, Br, $CF_3$ und/oder CN substituiert sein kann,
A $-(CH_2)_4-$, $-(CH_2)_3-CO-$ oder $-CO-(CH_2)_2-CO-$,
$R^1$ H oder Methyl,
$R^2$ H oder zusammen mit $R^3$ eine C–C-Bindung,
$R^3$ H oder zusammen mit $R^2$ eine C–C-Bindung,
$R^4$ und $R^5$ jeweils H, Alkyl oder Phenyl und
Ar eine unsubstituierte oder ein- oder zweifach durch Alkyl, F, Cl, Br, J und/oder $CF_3$ substituierte Phenylgruppe
bedeuten und
worin die Alkylgruppen jeweils 1–4 C-Atome besitzen,
sowie deren physiologisch unbedenklichen Säureadditionssalzen, dadurch gekennzeichnet, dass man eine Verbindung der allgemeinen Formel II

Ind-A-$X^1$ II

worin
$X^1$ X oder $NH_2$ und
X Cl, Br, J, OH oder eine reaktionsfähig funktionell abgewandelte OH-Gruppe bedeuten und
Ind und A die angegebenen Bedeutungen haben, mit einer Verbindung der allgemeinen Formel III

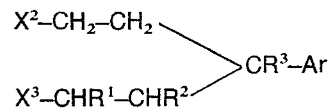 III

worin

$X^2$ und $X^3$ gleich oder verschieden sein können und, falls $X^1 = NH_2$ ist, jeweils X, andernfalls zusammen NH bedeuten und
$R^1$, $R^2$, $R^3$ und Ar die angegebenen Bedeutungen haben
umsetzt oder dass man ein Isonitril der allgemeinen Formel IV

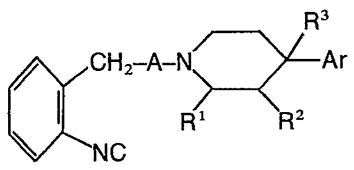 IV

worin
$R^1$, $R^2$, $R^3$, A und Ar die angegebenen Bedeutungen haben
mit Hilfe von Lithium-2,2,6,6-tetramethylpiperidid cyclisiert oder dass man ein Pyridiniumsalz der allgemeinen Formel V

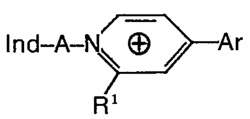 $An^{\ominus}$ V

worin
$An^{\ominus}$ ein Anion einer starken Säure bedeutet und Ind, A, $R^1$ und Ar die angegebenen Bedeutungen haben
mit einem reduzierenden Mittel behandelt und dass man gegebenenfalls in einer erhaltenen Verbindung eine oder mehrere CO-Gruppen zu $CH_2$-Gruppen reduziert und/oder eine Doppelbindung hydriert und/oder eine freie NH-Gruppe im Indolring alkyliert und/oder dass man eine erhaltene Base der Formel I durch Behandeln mit einer Säure in eines ihrer physiologisch unbedenklichen Säureadditionssalze umwandelt.

**Claims**
for the Contracting states: BE, CH, DE, FR, GB, IT, NL, SE

1. Indolealkylamines of the general formula I

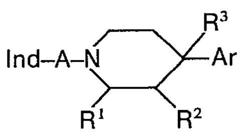 I

wherein Ind is a $1-R^4-2-R^5$-indol-3-yl radical which in the benzene ring can be monosubstituted to trisubstituted by alkyl, O-alkyl, S-alkyl, OH, F, Cl, Br, $CF_3$ and/or CN, A is $-CH_2)_4-$, $-(CH_2)_3-CO-$ or $-CO-(CH_2)_2-CO-$, $R^1$ is H or methyl, $R^2$ is H or together with $R^3$ is a C–C bond, $R^3$ is H or together with $R^2$ is a C–C bond, $R^4$ and $R^5$ are each H, alkyl or phenyl and Ar is a phenyl group which is unsubstituted or monosubstituted or disubstituted by alkyl, F, Cl, Br, I and/or $CF_3$, and wherein the alkyl groups in each case possess 1–4 C atoms, and their physiologically acceptable acid addition salts.

2. 3-[4-(4-Phenyl-3,4-dehydro-1-piperidyl)-butyl]-indole.

3. Process for the preparation of indolealkylamines of the general formula I according to Claim 1 and their physiologically acceptable acid addition salts, characterised in that a compound of the general formula II

$$Ind–A–X^1 \qquad II$$

wherein $X^1$ is X or $NH_2$ and X is Cl, Br, I, OH or a reactively functionnally modified OH group and Ind and A have the indicated meanings, is reacted with a compound of the general formula III

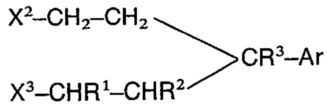 III

wherein $X^2$ and $X^3$ can be identical or different and, if $X^1$ is $NH_2$, are in each case X and otherwise together are NH, and $R^1$, $R^2$, $R^3$ and Ar have the indicated meanings, or in that an isonitrile of the general formula IV

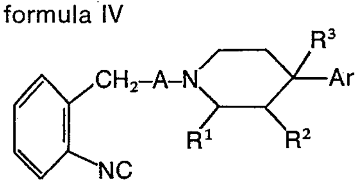 IV

which in the benzene ring can be monosubstituted to trisubstituted by alkyl, O-alkyl, S-alkyl, OH, F, Cl, Br, $CF_3$ and/or CN and
wherein $R^1$, $R^2$, $R^3$, A and Ar have the indicated meanings, is cyclised with the aid of lithium 2,2,6,6-tetramethylpiperidide, or in that a pyridinium salt of the general formula V

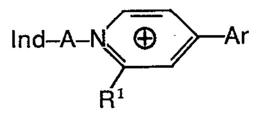 $An^{\ominus}$ V

wherein $An^{\ominus}$ is an anion of a strong acid and Ind, A, $R^1$ and Ar have the indicated meanings, is treated with a reducing agent and/or in that, optionally, in a compound of formula I one or two CO groups are reduced to $CH_2$ groups and/or a double bond is hydrogenated and/or a free NH group in the indole ring is alkylated and/or an alkoxy group is split with formation of a hydroxy group and/or in that a base of the formula I is converted by treatment with an acid to one of its physiologically acceptable acid addition salts.

4. Process for the preparation of pharmaceutical formulations, characterised in that a compound of the formula I and/or one of its physiologically acceptable acid addition salts is brougth into a suitable dosage form together with at least one solid, liquid or semi-liquid excipient or auxiliary and optionally in combination with one or more other active compounds.

5. Pharmaceutical formulation, characterised in that it contains at least one compound of the general formula I and/or one of its physiologically acceptable acid addition salts.

**Claims**
for the Contracting state: AT

1. Process for the preparation of indolealkylamines of the general formula I

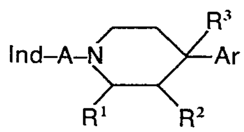 I

wherein Ind is a 1-$R^4$-2-$R^5$-indol-3-yl radical which in the benzene ring can be monosubstituted to trisubstituted by alkyl, O-alkyl, S-alkyl, OH, F, Cl, Br, $CF_3$ and/or CN, A is $-(CH_2)_4-$, $-(CH_2)_3-CO-$ or $CO-(CH_2)_2-CO-$, $R^1$ is H or methyl, $R^2$ is H or together with $R^3$ is a C–C bond, $R^3$ is H or together with $R^2$ is a C–C bond, $R^4$ and $R^5$ are each H, alkyl or phenyl and Ar is a phenyl group which is unsubstituted or monosubstituted or disubstituted by alkyl, F, Cl, Br, I and/or $CF_3$, and wherein the alkyl groups in each case possess 1–4 C atoms, and their physiologically acceptalbe acid addition salts, characterised in that a compound of the general formula II

$$Ind-A-X^1 \qquad II$$

wherein $X^1$ is X or $NH_2$ and X is Cl, Br, I, OH or a reactively functionnaly modified OH group and Ind and A have the indicated meanings, is reacted with a compound of the general formula III

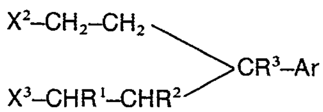 III

wherein $X^2$ and $X^3$ can be identical or different and, if $X^1$ is $NH_2$, are in each case X and otherwise together are NH, and $R^1$, $R^2$, $R^3$ and Ar have the indicated meanings, or in that an isonitrile of the general formula IV

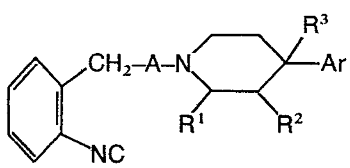 IV

which in the benzene ring can be monosubstituted to trisubstituted by alkyl, O-alkyl, S-alkyl, OH, F, Cl, Br, $CF_3$ and/or CN and wherein $R^1$, $R^2$, $R^3$, A and Ar have the indicated meanings, is cyclised with the aid of lithium 2,2,6,6-tetramethylpiperidide, or in that a pyridinium salt of the general formula V

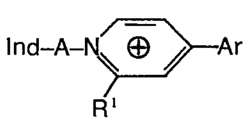 $An^{\ominus}$ V

wherein $An^{\ominus}$ is an anion of a strong acid and Ind, A, $R^1$ and Ar have the indicated meanings, is treated with a reducing agent and/or in that, optionally, in a compound of formula I one or two CO groups are reduced to $CH_2$ groups and/or a double bond is hydrogenated and/or a free NH group in the indole ring is alkylated and/or an alkoxy group is split with formation of a hydroxy group and/or in that a base of the formula I is converted by treatment with an acid to one of its physiologically acceptable acid addition salts.

2. Process for the preparation of indolealkylamines of the general formula I

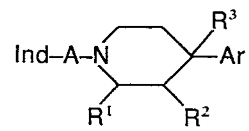 I

wherein Ind is a 1-$R^4$-2-$R^5$-indol-3-yl radical which in the benzene ring can be monosubstituted to trisubstituted by alkyl, O-alkyl, S-alkyl, Cl, Br, $CF_3$ and/or CN, A is $-(CH_2)_4-$, $-(CH_2)_3-CO-$ or $-CO-(CH_2)_2-CO-$, $R^1$ is H or methyl, $R^2$ is H or together with $R^3$ is a C–C bond, $R^3$ is H or together with $R^2$ is a C–C bond, $R^4$ and $R^5$ are each H, alkyl or phenyl and Ar is a phenyl group which is unsubstituted or monosubstituted or disubstituted by alkyl, F, Cl, Br, I and/or $CF_3$, and wherein the alkyl groups in each case possess 1–4 C atoms, and their physiologically acceptable acid addition salts, characterised in that a compound of the general formula II

$$Ind-A-X^1 \qquad II$$

wherein $X^1$ is X or $NH_2$ and X is Cl, Br, I, OH or a reactively functionally modified OH group and Ind and A have the indicated meanings, is reacted with a compound of the general formula III

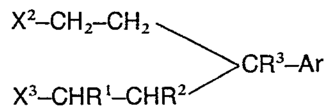 III

wherein $X^2$ and $X^3$ can be identical or different and, if $X^1$ is $NH_2$, are in each case X and otherwise together are NH, and $R^1$, $R^2$, $R^3$ and Ar have the indicated meanings, or in that an isonitrile of the general formula IV

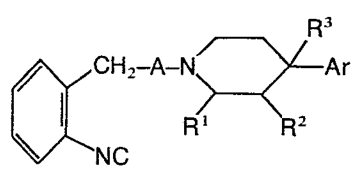 IV

wherein $R^1$, $R^2$, $R^3$, A and Ar have the indicated meanings, is cyclised with aid of lithium 2,2,6,6-tetramethylpiperidide, or in that a pyridinium salt of the general formula V

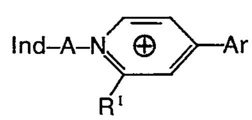 $An^{\ominus}$ V

where $An^{\ominus}$ is an anion of a strong acid and Ind, A, $R^1$ and Ar have the indicated meanings, is treated with a reducing agent and in that, optionally, in a resulting compound one or more CO groups are reduced to $CH_2$ groups and/or a double bond

is hydrogenated and/or a free NH group in the indole ring is alkylated and/or in that a resulting base of the formula I is converted by treatment with an acid to one of its physiologically acceptable acid addition salts.

**Revendications**
pour les Etats contractants: BE, CH, DE, FR, GB, IT, NL, SE

1. Indolalcoylamines de formule générale

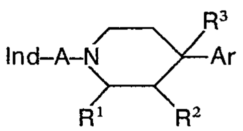

I

où
Ind représente un radical 1-$R^4$-2-$R^5$-indol-3-yle, qui peut être mono- à tri-substitué par un groupe alcoyle, un groupe O-alcoyle, un groupe S-alcoyle, OH, F, Cl, Br, $CF_3$ et/ou CN,
A représente $-(CH_2)_4$, $-(CH_2)_3-CO-$ ou $-CO-(CH_2)_2-CO-$,
$R^1$ H ou un groupe méthyle,
$R^2$ H ou ensemble avec $R^3$ une liaison C–C
$R^3$ H ou ensemble avec $R^2$ une liaison C–C,
$R^4$ et $R^5$ chacun H, un groupe alcoyle ou un groupe phényle
et
Ar un groupe phényle non substitué ou mono- ou di-substitué par un groupe alcoyle, F, Cl, Br, I et/ou $CF_3$ et où les groupes alcoyle possèdent chacun de 1 à 4 atomes de carbone,
ainsi que leurs sels d'addition acides physiologiquement acceptables.

2. 3-[4-(4-phényl-3,4-déhydro-1-pipéridyl)-butyl]-indole.

3. Procédé de préparation d'indolalcoylamines de formule générale I selon la revendication 1 ainsi que de leurs sels d'addition acides physiologiquement acceptables, caractérisé en ce qu'on fait réagir un composé de formule générale II

$$Ind-A-X^1 \qquad II$$

$X^1$ représente X ou $NH_2$ et
X Cl, Br, I, OH ou un groupe OH réactif fonctionnellement modifié et Ind et A ont les significations mentionnées,
avec un composé de formule générale III

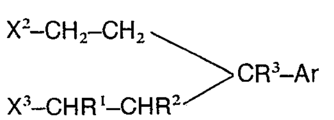

III

où
$X^2$ et $X^3$ peuvent être semblables ou différents et si $X^1 = NH_2$, représentent chacun X, sinon, ensemble, NH et $R^1$, $R^2$, $R^3$ et Ar ont les significations données
ou en ce qu'on cyclise un isonitrile de formule générale IV

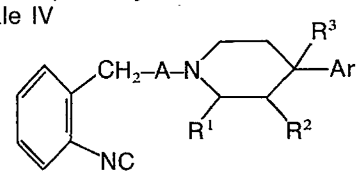

IV

où
le noyau benzénique peut être mono- à tri-substitué par un groupe alcoyle, O-alcoyle, S-alcoyle, OH, F, Cl, Br, $CF_3$ et/ou CN et
$R^1$, $R^2$, $R^3$, A et Ar ont les significations données à l'aide de 2,2,6,6-tétraméthylpipéridure de lithium, ou en ce qu'on traite un sel de pyridinium de formule générale V

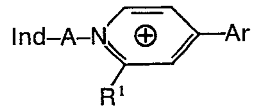

$An^{\ominus}$ V

où
$An^{\ominus}$ représente un anion d'un acide fort et
Ind, A, $R^1$ et Ar ont les significations mentionnées, avec un agent réducteur,
et/ou en ce qu'éventuellement on réduit dans un composé de formule I un ou deux groupes CO en groupes $CH_2$ et/ou en ce qu'on hydrogène une double liaison et/ou en ce qu'on alcoyle un groupe NH libre dans le noyau indole et/ou en ce qu'on sépare un groupe alcoxy avec formation d'un groupe hydroxy et/ou en ce qu'on transforme une base de formule I par traitement avec un acide en l'un de ses sels d'addition acides physiologiquement acceptables.

4. Procédé de préparation de préparations pharmaceutiques, caractérisé en ce qu'on amène sous une forme posologique appropriée un composé de formule I et/ou l'un de ses sels d'addition acides physiologiquement acceptables avec au moins un support ou adjuvant solide, liquide ou semi-liquide et éventuellement en combinaison avec une ou plusieurs autres substances actives.

5. Préparation pharmaceutique, caractérisé en ce qu'elle contient au moins un composé de formule générale I et/ou un de ses sels d'addition acides physiologiquement acceptables.

**Revendications**
pour l'Etat contractant: AT

1. Procédé de préparation d'indolalcoylamines de formule générale

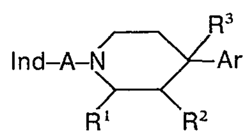

I

où
Ind représente un radical 1-$R^4$-2-$R^5$-indol-3-yle, qui peut être mono- à tri-substitué par un groupe alcoyle, un groupe O-alcoyle, un groupe S-alkoyle, OH, F, Cl, Br, $CF_3$ et/ou CN,
A représente $-(CH_2)_4$, $-(CH_2)_3-CO-$ ou $-CO-(CH_2)_2-CO-$,
$R^1$ H ou un groupe méthyle,
$R^2$ H ou ensemble avec $R^3$ une liaison C–C
$R^3$ H ou ensemble avec $R^2$ une liaison C–C,
$R^4$ et $R^5$ chacun H, un groupe alcoyle ou un groupe phényle
et
Ar un groupe phényle non substitué ou mono- ou di-substitué par un groupe alcoyle, F, Cl, Br, I et/ou $CF_3$ et où les groupes alcoyle possèdent

chacun de 1 à 4 atomes de carbone, ainsi que de leurs sels d'addition acides physiologiquement acceptables, caractérisé en ce qu'on fait réagir un composé de formule générale II

Ind–A–$X^1$ II

où

$X^1$ représente X ou $NH_2$ et

X Cl, Br, I, OH ou un groupe OH réactif fonctionnellement modifié et Ind et A ont les significations mentionnées,

avec un composé de formule générale III

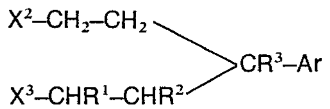
III

où

$X^2$ et $X^3$ peuvent être semblables ou différents et si $X^1$ = $NH_2$, représentent chacun X, sinon, ensemble NH et $R^1$, $R^2$, $R^3$ et Ar ont les significations données

ou en ce qu'on cyclise un isonitrile de formule générale IV

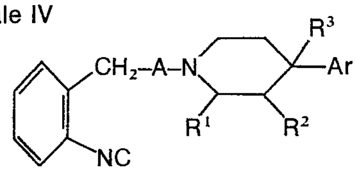
IV

où

le noyau benzénique peut être mono- à tri-substitué par un groupe alcoyle, O-alcoyle, S-alcoyle, OH, F, Cl, Br, $CF_3$ et/ou CN et

$R^1$, $R^2$, $R^3$, A et Ar ont les significations données à l'aide de 2,2,6,6-tétraméthylpipéridure de lithium, ou en ce qu'on traite un sel de pyridinium de formule générale V

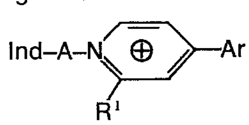
$An^{\ominus}$ V

où

$An^{\ominus}$ représente un anion d'un acide fort et

Ind, A, $R^1$ et Ar ont les significations mentionnées, avec un agent réducteur,

et/ou en ce qu'éventuellement on réduit dans un composé de formule I un ou deux groupes CO en groupes $CH_2$ et/ou en ce qu'on hydrogène une double liaison et/ou en ce qu'on alcoyle un groupe NH libre dans le noyau indole et/ou en ce qu'on sépare un groupe alcoxy avec formation d'un groupe hydroxy et/ou en ce qu'on transforme une base de formule I par traitement avec un acide en l'un de ses sels d'addition acides physiologiquement acceptables.

2. Procédé de préparation d'indolalcoylamines de formule générale

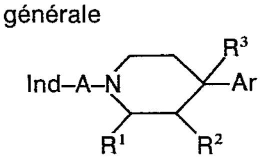
I

où

Ind représente un radical 1-$R^4$-2-$R^5$-indol-3-yle, qui peut être mono- à tri-substitué par un groupe alcoyle, un groupe O-alcoyle, un groupe S-alcoyle, F, Cl, Br, $CF_3$ et/ou CN,

A représente $-(CH_2)_4$, $-(CH_2)_3-CO-$ ou $-CO-(CH_2)_2-CO-$,

$R^1$ H ou un groupe méthyle,

$R^2$ H ou ensemble avec $R^3$ une liaison C–C

$R^3$ H ou ensemble avec $R^2$ une liaison C–C,

$R^4$ et $R^5$ chacun H, un groupe alcoyle ou un groupe phényle

et

Ar un groupe phényle non substitué ou mono- ou di-substitué par un groupe alcoyle, F, Cl, Br, I et/ou $CF_3$ et où les groupes alcoyle possèdent chacun de 1 à 4 atomes de carbone,

ainsi que de leurs sels d'addition acides physiologiquement acceptables, caractérisé en ce qu'on fait réagir un composé de formule générale II

Ind–A–$X^1$ II

où

$X^1$ représente X ou $NH_2$ et

X Cl, Br, I, OH ou un groupe OH réactif fonctionnellement modifié et Ind et A ont les significations mentionnées,

avec un composé de formule générale III

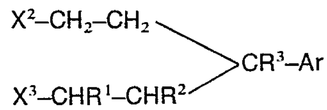
III

où

$X^2$ et $X^3$ peuvent être semblables ou différents et si $X^1$ = $NH_2$, représentent chacun X, sinon, ensemble, NH et $R^1$, $R^2$, $R^3$ et Ar ont les significations données

ou en ce qu'on cyclise un isonitrile de formule générale IV

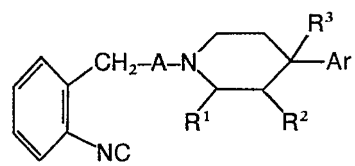
IV

où

$R^1$, $R^2$, $R^3$, A et Ar ont les significations données à l'aide de 2,2,6,6-tétraméthylpipéridure de lithium, ou en ce qu'on traite un sel de pyridinium de formule générale V

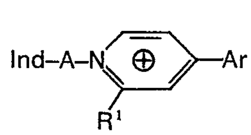
$An^{\ominus}$ V

où

$An^{\ominus}$ représente un anion d'un acide fort et

Ind, A, $R^1$ et Ar ont les significations mentionnées, avec un agent réducteur,

et en ce qu'éventuellement on réduit dans un composé obtenu un ou plusieurs groupes CO en groupes $CH_2$ et/ou en ce qu'on hydrogène une double liaison et/ou en ce qu'on alcoyle un groupe NH libre dans le noyau indole et/ou en ce qu'on transforme une base obtenue de formule I par traitement avec un acide en l'un de ses sels d'addition acides physiologiquement acceptables.